Europäisches Patentamt

European Patent Office

Office européen des brevets

(11) Numéro de publication: **0 528 135 A1**

# DEMANDE DE BREVET EUROPEEN

(21) Numéro de dépôt: **92110344.6**

(22) Date de dépôt: **19.06.92**

(51) Int. Cl.5: **A61K 31/20**

(30) Priorité: **20.08.91 CH 2450/91**

(43) Date de publication de la demande:
**24.02.93 Bulletin 93/08**

(84) Etats contractants désignés:
**AT BE CH DE DK ES FR GB GR IT LI LU MC
NL PT SE**

(71) Demandeur: **SOCIETE DES PRODUITS NESTLE
S.A.
Case postale 353
CH-1800 Vevey(CH)**

(72) Inventeur: **Borel, Christian
5, Rte d'Echallens
CH-1032 Romanel-s/Lausanne(CH)**
Inventeur: **Guichardant, Michel
32, Bd de la Forêt
CH-1009 Pully(CH)**

(54) **Utilisations de l'acide octadécatriène-9(Z),12(Z),15(Z)-ynoique-6.**

(57) La présente invention a pour objet l'utilisation de l'acide octadécatriène-9(Z),12(Z),15(Z)-ynoïque-6 en tant que substance thérapeutiquement active.

La présente invention a trait à l'utilisation de l'acide octadécatriène-9(Z),12(Z),15(Z)-ynoïque-6, que l'on désignera, dans la suite de la présente description, par le terme "dicranine".

La dicranine est un acide gras polyinsaturé, dont l'ester méthylique peut être extrait de la mousse Ceratodon purpureus, ainsi que le montre l'article de J. Gellerman et al. publié dans Biochemistry (1977), vol.16, 7, 1258-1262.

Il a été trouvé que, de manière surprenante, la dicranine présentait des propriétés thérapeutiques, et, en particulier, des propriétés anti-agrégantes et anti-inflammatoires, ainsi qu'anti-bactériennes.

Ainsi la présente invention concerne-t-elle l'utilisation de la dicranine en tant que substance thérapeutiquement active.

La présente invention concerne en particulier l'utilisation de la dicranine en tant qu'anti-agrégant, anti-inflammatoire et anti-bactérien.

La présente invention concerne également une composition pharmaceutique comprenant, à titre de substance thérapeutiquement active, la dicranine ainsi qu'un excipient pharmaceutiquement neutre.

Ladite composition pharmaceutique peut se présenter, par exemple, sous forme d'une solution injectable, ou sous forme de comprimé.

La présente invention concerne aussi une composition cosmétique comprenant la dicranine.

Ladite composition pharmaceutique peut se présenter, par exemple, sous forme d'une crème, d'un onguent ou d'une lotion.

Les exemples suivants illustrent la présente invention plus en détail.

Dans ces exemples, on utilise les abréviations suivantes:

- 13-HODE      acide hydroxy-13-octadécadiénoïque
- 12-HETE      acide hydroxy-12-eicosatétraénoïque
- 5-HETE       acide hydroxy-5-eicosatétraénoïque
- HHT           acide hydroxy-12-heptadécatriénoïque
- 12-HPETE     acide hydroperoxy-12-eicosatétraénoïque
- $LTB_4$          acide (5S,12R)-5,12-dihydroxy-6,14-cis-8,10-trans-eicosatétraénoïque
- $PGG_2$         acide hydroperoxy-15-peroxydoprosta-9$\alpha$,11$\alpha$-diénoïque-5,13
- $PGH_2$         acide hydroxy-15-peroxydoprosta-9$\alpha$,11$\alpha$--diénoïque-5,13

L'exemple 1 décrit un procédé de synthèse de la dicranine.

Les exemples 2 à 6 mettent en évidence les propriétés anti-inflammatoire, anti-agrégante et anti-bactérienne de la dicranine.

Exemple 1

a) On ajoute à 200 ml de NH3 liquide, 50 mg de Fe(NO3)3 et 10,9 g de sodium métallique.

On agite pendant 1 heure, à température ambiante, puis l'on ajoute, goutte à goutte, 50 ml de propargyloxytetrahydropyrane. On agite pendant encore 1 heure et l'on ajoute 30 ml d'iodure d'éthyle.

On continue l'agitation pendant 12 heures, de manière à laisser l'ammoniac s'échapper.

On ajoute alors de l'acide sulfurique jusqu'à obtention d'un pH de environ 4, puis l'on extrait le mélange à l'éther.

On récupère la phase ethérée, et l'on évapore le solvant. Le résidu est dissous dans 50 ml de méthanol, on lui ajoute 10 ml d'acide sulfurique à 25%, on laisse agiter pendant 1 heure, puis on dilue le mélange avec de l'eau.

Le mélange dilué est alors extrait à l'éther.

On récupère la phase ethérée, qui est séchée sur du sulfate de magnésium, puis l'on évapore le solvant sous pression réduite.

On distille le résidu et on obtient 22 g de pentyne-2-ol-1.

b) On ajoute à une solution de 10,5 g de pentyne-2-ol-1 dans 150 ml d'éther, 0,5 ml de pyridine, puis, goutte-à-goutte, 4,2 ml de tribromure de phosphore.

On chauffe le mélange à reflux pendant 3 heures, puis on lui ajoute de la glace.

Le mélange est alors extrait à l'éther.

On lave la phase éthérée avec une solution de carbonate de potassium, puis on sèche sur du sulfate de magnésium et on évapore le solvant sous pression réduite.

On distille le résidu et on obtient 9,75 g de bromo-1-pentyne-2.

c) On ajoute, sous agitation, 25,5 g de bromure d'éthyle à une solution de 100 ml de THF contenant 5,0 g de magnésium.

Le mélange est refroidit jusqu'à 0°C, et on lui ajoute doucement une solution de 6,0 g d'alcool propargylique dans 10 ml de tétrahydrofurane (THF).

Le mélange est agité à 20°C pendant 2 heures, puis refroidit jusqu'à 0°C.

On ajoute alors 0,5 g de chlorure cuivreux, puis une solution de 9,7 g de bromo-1-pentyne-2 dans 15 ml de THF.

On chauffe le mélange à reflux pendant 19 heures, on ajoute alors un excès de chlorure cuivreux, et l'on continue le chauffage pendant 5 heures.

On ajoute un mélange glace-acide sulfurique, et l'on extrait le mélange résultant à l'aide d'éther.

On lave la phase éthérée avec une solution de carbonate de potassium, on la sèche sur sulfate de magnésium et on évapore le solvant sous pression réduite.

On distille le résidu et l'on obtient 6,8 g de octadiyne-2,5-ol-1.

d) De manière analogue à la réaction décrite sous b), on fait réagir 6,5 g de octadiyne-2,5-ol-1 et 2 ml de tribromure de phosphore dans 75 ml d'éther, de manière à obtenir 6,3 g de bromo-1-octadiyne-2,5.

e) De manière analogue à la réaction décrite sous c), on ajoute 3,6 ml d'alcool propargylique et 6,0 g de bromo-1-octadiyne-2,5 à 9,6 ml de bromure d'éthyle et 2,8 g de magnésium, dans 50 ml de THF.

On obtient 4,5 g d'undecatriyne-2,5,8-ol-1, sous forme d'un solide blanc.

f) On ajoute à une solution de 2,0 g de undecatriyne-2,5,8-ol-1 dans 30 ml d'acétate d'éthyle, 0,5 ml de pyridine et 1,0 g de catalyseur de Lindlar. On place le mélange sous hydrogène, à 1 bar, pendant 24 heures.

On récupère le catalyseur par filtration et on évapore le solvant sous pression réduite.

Par distillation du résidu, on obtient 0,8 g d'undecatriene-2,5,8-ol-1.

g) De manière analogue à la réaction décrite sous b), on chauffe à reflux pendant 30 minutes un mélange de 0,8 g d'undecatriene-2,5,8-ol-1 et de 0,3 ml de tribromure de phosphore dans 20 ml d'éther. Après distillation, on obtient 0,52 g de bromo-1-undecatriène-2,5,8.

h) On ajoute du brome à une solution de 5,0 g d'acide heptène-6-oïque dans 70 ml d'éther, de manière à obtenir le composé dibromé (contrôlé par RMN).

On ajoute à ce mélange une solution de 5 g de sodium dans 150 ml de $NH_3$ liquide, et l'on agite pendant 12 heures, de manière à ce que l'ammoniac s'échappe.

On ajoute alors 10,0 g de chlorure d'ammonium solide et 200 ml d'eau.

On acidifie le mélange par ajout d'HCl 6N, puis on l'extrait avec de l'éther.

On lave la phase éthérée à l'eau, puis on la sèche sur du sulfate de magnésium, et l'on évapore le solvant sous pression réduite.

Après distillation du résidu, on obtient 3,5 g d'acide heptyne-6-oïque.

i) A une solution de bromure d'éthylmagnésium, préparée à partir de 0,21 g de magnésium et 0,97 g de bromure d'éthyle dans 50 ml de THF, on ajoute, goutte-à-goutte, 550 mg d'acide heptyne-6-oïque dans 5 ml de THF.

On agite le mélange à 20°C pendant 1 heure, on ajoute 20 mg de CuCN, puis 500 mg de bromo-1-undécatriène-2,5,8. Le mélange est chauffé à reflux pendant 6 heures, refroidi, puis additionné de glace et acidifié à l'aide d'HCl dilué.

On extrait le mélange à l'éther, on récupère et sèche la phase éthérée sur du sulfate de magnésium, on évapore les solvants résiduels sous pression, et l'on obtient une huile jaune.

Par chromatographie sur colonne et distillation, on obtient 260 mg d'une huile incolore, que l'on identifie par RMN du carbone et du proton, et par spectrométrie de masse, comme étant l'acide octadécatriène-9-(Z),12(Z),-15(Z)-ynoïque-6.


Exemple 2

Cet exemple teste l'effet de la dicranine sur la lipoxygénase et la cyclooxygénase des plaquettes sanguines humaines isolées.

On isole des plaquettes à partir de sang humain prélevé sur un anti-coagulant de type ACD (Lagarde et al, Thrombos. Res. (1979), 17, 581-588).

On ajoute de la dicranine solubilisée dans l'éthanol aux plaquettes en suspension dans un tampon physiologique (Hepes), à raison de environ $3.10^5$ cellules/$\mu$l, de manière à obtenir une concentration de $10^{-4}$ ou $10^{-6}$ M en dicranine.

On laisse incuber les plaquettes à 37°C pendant 10 minutes, puis on les stimule par ajout d'une solution d'acide arachidonique de manière à obtenir une concentration en acide arachidonique de $10^{-5}$ M, et on laisse à nouveau incuber pendant 10 minutes à 37°C.

On acidifie jusqu'à pH3 par addition d'HCl 3N.

On extrait les lipides totaux à l'aide d'éther éthylique, et l'on en sépare les acides gras monohydroxylés, par chromatographie sur couche mince, selon la méthode décrite par Guichardant et al., Biochem. Biophys.

Acta, (1985), 836, 210-214.

On mesure les acides gras monohydroxylés par chromatographie liquide à haute performance (HPLC) et on les quantifie par comparaison avec le standard 13-HODE, en faisant l'hypothèse qu'ils ont tous le même coefficient d'extinction molaire à $3.10^4$ $M^{-1}$ $cm^{-1}$.

Lors de l'activation plaquettaire, deux voies métaboliques de l'acide arachidonique sont activées:

- la voie de la lipoxygénase, qui conduit à la formation du 12-HPETE, qui est réduit en 12-HETE,
- la voie de la cyclooxygénase, qui forme les métabolites responsables de l'agrégation plaquettaire, à savoir:
  - les endoperoxydes $PGG_2$, $PGH_2$ et le thromboxane $A_2$ ($TXA_2$) qui se stabilise en $TXB_2$
  - les prostaglandines de la série 2 et
  - un acide gras monohydroxylé, le HHT.

Le dosage du 12-HETE permet d'apprécier l'activité de la lipoxygénase.

Le dosage du HHT permet d'apprécier l'activité de la cyclooxygénase.

On obtient les résultats suivants:

| Concentration en dicranine | Effet Observé visuellement | Augmentation du 12-HETE par rapport au témoin | Diminution du HHT par rapport au témoin |
|---|---|---|---|
| Témoin | agrégation | -- | -- |
| $10^{-6}$ M | faible inhibition | 40% | 18% |
| $10^{-4}$ M | inhibition de l'agrégation des plaquettes | 654% | 31% |

Pour une concentration en dicranine de $10^{-4}$ M, on observe une très forte activation de la voie lipoxygénase, et la diminution de la voie cyclooxygénase.

Ce résultat montre que l'augmentation du 12-HETE n'est pas reliée à une plus grande disponibilité de l'acide arachidonique, moins bien utilisé par la cyclooxygénase.

On remarque donc que la dicranine a pour effet d'augmenter le 12-HETE. Or l'on sait que le 12-HETE est impliqué dans la régulation de processus immunologiques.

On peut donc penser que la dicranine possède un effet immunologique.

Pour une concentration en dicranine de $10^{-6}$ M, le même effet est observé, mais plus faiblement.


Exemple 3

Cet exemple teste les propriétés anti-agrégantes de la dicranine sur des plaquettes sanguines humaines isolées.

Le test utilisé est la méthode turbidimétrique de Born, décrite dans Nature (1962), 194, 927-929.

Plusieurs inducteurs, agissant chacun spécifiquement à différents niveaux de l'action de l'acide arachidonique, ont été utilisés:

- la thrombine, qui prend en compte tout le mécanisme enzymatique conduisant à l'agrégation,
- l'acide arachidonique, substrat direct de la cyclooxygénase qui forme les médiateurs responsables de l'agrégation, à savoir les endoperoxydes $PGG_2$, $PGH_2$ et le thromboxane $A_2$ ($TXA_2$),
- le 9-méthano-$PGH_2$, (analogue structurel de $PGH_2$), référence U46619) qui à lui seul, provoque l'agrégation.

Les résultats suivants ont été obtenus, pour une concentration en dicranine de $10^{-4}$ M:

1. Sans incubation préalable de la dicranine.

Lorsque l'on utilise comme inducteur la thrombine (à raison de 0,1 unité/ml) ou l'acide arachidonique (à raison de $5.10^{-6}$ M), on observe une agrégation normale des plaquettes (pas d'inhibition).

Lorsque l'on utilise comme inducteur, le 9-méthano-$PGH_2$ (à raison de 1 $\mu$g/ml), on observe une inhibition totale de l'agrégation.

On peut donc en déduire que la dicranine n'agit pas au niveau des enzymes phospholipase ou cyclooxygénase; elle semble, par contre, agir directement sur le site récepteur de $PGH_2/TXA_2$, pour inhiber l'agrégation.

Si l'on ajoute une seconde dose de 9-méthano-$PGH_2$ (1 $\mu$g/ml), on n'observe pas d'agrégation.

Le processus d'inhibition semble donc irréversible.

2. Avec incubation de la dicranine.

Après 10 minutes d'incubation à 37°C, on observe une faible inhibition de l'agrégation, lorsqu'on utilise la thrombine comme inducteur.

Après 20 minutes d'incubation à 37°C, on observe une inhibition beaucoup plus importante de l'agrégation avec la thrombine.

Une explication peut être que l'inhibition de l'agrégation nécessite la formation d'un métabolite de la dicranine, cette formation s'effectuant selon une cinétique lente.

Les résultats sont résumés dans le tableau ci-dessous:

| Inducteurs | Taux d'agrégation (%) | |
| --- | --- | --- |
| | Témoin | Dicranine $(10^{-4}$ M) |
| **Thrombine** | | |
| . sans incubation | 65 | 70 |
| . 10 min | 70 | 69 |
| . 20 min | 68 | 44 |
| **Acide arachidonique** | | |
| . sans incubation | 70 | 70 |
| **9-méthano-PGH$_2$** | | |
| . sans incubation | 66 | 0 |

Cet effet peut également provenir du fait que la synthèse du 12-HETE, connu comme inhibiteur de l'agrégation plaquettaire, est considérablement augmentée en présence de dicranine. Il en est de même pour la synthèse du 12 -HPETE, son précurseur.

On peut conclure du présent exemple que la dicranine, en une concentration d'au moins $10^{-4}$ M, et après incubation, présente des propriétés anti-agrégantes vis-à-vis des plaquettes sanguines humaines.

De plus, la dicranine semble ne pas agir directement, mais plutôt par ses métabolites (acides gras monohydroxylés).

Exemple 4

Un des métabolites principaux de la dicranine a été isolé par HPLC. Il s'agit de l'acide hydroxy-13-octadécatriène-9(Z),12(Z),15(Z)-ynoïque-6.

Les propriétés anti-agrégantes de ce métabolite ont été étudiées, selon l'exemple 3, en utilisant la thrombine comme inducteur, à raison de 0,1 unité par ml.

A une concentration en métabolite à $10^{-6}$ M, on observe, sans incubation ou après 10 minutes d'incubation, une inhibition lente de l'agrégation des plaquettes, induite par la thrombine.

Exemple 5

Cet exemple a pour but de tester les effets de la dicranine sur les leucocytes humains isolés.

On isole des leucocytes humains selon la méthode décrite par Guichardant et al., Biochem. J. (1988), 256, 79-883.

Les leucocytes sont ensuite éventuellement incubés, à 37°C, pendant 30 minutes, en présence de dicranine à une concentration de $10^{-4}$ ou $10^{-6}$ M, dans l'éthanol, puis les leucocytes éventuellement incubés sont stimulés par ajout d'acide arachidonique, à une concentration de $10^{-5}$ M, et de calcium ionophore (Oven A23187-Sigma), à une concentration de $2.10^{-6}$ M, et laissés à 37°C pendant 10 minutes. On acidifie alors jusqu'à pH3 par addition d'acide chlorhydrique 3N.

On extrait les lipides totaux à l'aide d'un mélange éthanol/chloroforme 1:2, puis on sépare les lipides extraits par chromatographie bidimentionnelle sur couche mince.

On développe les acides gras monohydroxylés dans la première dimension au moyen d'un mélange solvant hexane/éther éthylique/acide acétique glacial en proportion volumique 59/40/1, les acides gras dihydroxylés restant à l'origine de la plaque. On récolte les acides gras monohydroxylés de la plaque, ainsi que le 13-HODE, on les extrait avec l'éther éthylique, on évapore le solvant sous azote, puis on les sépare par HPLC en phase inverse avec un mélange solvant d'élution méthanol/acide acétique aqueux de pH3 en proportion volumique 73/27. On les détecte par spectroscopie UV à la longueur d'onde de 234 nm et on les quantifie par comparaison avec le standard 13-HODE en faisant l'hypothèse qu'ils ont tous le même coefficient d'extinction molaire à $3 \times 10^4$ $M^{-1}cm^{-1}$.

On développe ensuite les acides gras dihydroxylés restant à l'origine de la plaque dans la seconde dimension perpendiculairement à la première à l'aide d'un mélange solvant hexane/éther éthylique/acide acétique glacial en proportion volumique 25/74/1. Leurs récolte, extraction et séparation s'effectuent comme indiqué ci-dessus, sauf que le mélange solvant d'élution dans l'HPLC est méthanol/acide acétique aqueux de pH3 en proportion volumique 66/34. On utilise le PGB2 comme standard d'évaluation quantitative des acides gras dihydroxylés selon leur absorbance UV maximale respective (longueur d'onde 280 nm et coefficient d'extinction molaire $2,8 \times 10^4$ $M^{-1}cm^{-1}$ pour $PGB_2$; longueur d'onde 270 nm et coefficient d'extinction molaire $5 \times 10^4$ $M^{-1}cm^{-1}$ pour les dérivés dihydroxylés).

Il est connu que la stimulation des leucocytes par le calcium ionophore, en présence d'acide arachidonique, active la 5-lipoxygénase, et que cette activation se traduit par une synthèse importante de 5-HETE et de leucotriènes, tels que le $LTB_4$. C'est ce que l'on observe pour les leucocytes témoins n'ayant pas été incubés en présence de dicranine.

Dans le cas des leucocytes incubés en présence de $10^{-4}$ M de dicranine, on observe une inhibition de l'activation de la 5-lipoxygénase.

Cette inhibition entraîne une diminution importante de la synthèse du 5-HETE et du $LTB_4$. Cette diminution est de l'ordre de 50% pour leur synthèse, par comparaison avec le témoin, significative à 5% pour le $LTB_4$, selon le test apparié de Student-Fischer.

Dans le cas des leucocytes incubés en présence de $10^{-6}$ M de dicranine, on n'observe aucune inhibition de l'activation de la 5-lipoxygénase. A cette concentration, la dicranine n'est pas active.

La dicranine, à une concentration de $10^{-4}$ M, inhibe la synthèse des leucotriènes, qui sont les médiateurs de l'inflammation.

La dicranine présente donc des propriétés anti-inflammatoires.

Exemple 6

Ces exemples décrivent les propriétés anti-bactériennes de la dicranine vis-à-vis des bactéries suivantes:
- Bacillus stearothermophilus
- Bacillus cereus
- Bacillus subtilis
- Staphylococcus aureus
- Streptococcus faecalis

Exemple a

Sur une plaque de Pétri composé de 15 ml de gélose (PCA), on dépose 3 ml de gélose nutritive (SNA) inoculée à 1% avec une culture de la bactérie considérée préalablement incubée pendant 16-18 heures.

Sur un disque de papier filtre stérile, de 6,5 ou 13 mm de diamètre, on applique une certaine quantité de dicranine en solution éthanolique. Après évaporation du solvant, le disque est placé au centre de la plaque de Pétri.

On laisse incuber pendant 24 heures, à une température de 30-37°C pour les bactéries mésophiles, et à une température de 55°C pour les bactéries thermophiles. On observe visuellement que, dans une zone entourant le disque de papier, le microorganisme ne s'est pas développé.

On mesure le diamètre, en mm, de cette zone d'inhibition, et l'on obtient les résultats suivants:

| concentration en dicranine (μg/disque) | | | | | | | | |
|---|---|---|---|---|---|---|---|---|
| | 10 | 25 | 50 | 75 | 100 | 125 | 250 | 500 | 1000 |
| B. stearoth | 0 | 9 | 11 | 14 | 16 | 17 | 26 | 32 | 35 |
| B. cereus | 0 | 9 | 10 | 11 | 15 | 16 | 19 | 22 | 25 |
| B. subtilis | 0 | 8 | 11 | 13 | 13 | 14 | 16 | 19 | 20 |
| St. aureus | 0 | 10 | 11 | 12 | 16 | 17 | 21 | 22 | 23 |
| Str. faecalis | 6 | 10 | 12 | 13 | non déterminé | | | | |

On observe donc une inhibition du développement des bactéries à partir de 25 μg de dicranine par disque, voire même, pour Str.faecalis, à partir de 10 μg de dicranine/disque.

Exemple b

Dans une plaque à 96 puits, on place, par puits, 200 μl d'une infusion à base de cervelle et de coeur, contenant 0-200 μg/ml de dicranine sous la forme de son sel de sodium et on ajoute 50 μl d'une suspension aqueuse contenant $10^3$ à $10^5$ germes par ml.

On laisse incuber aux températures indiquées à l'exemple a.

Des échantillons (100 μl) sont prélevés au bout de 0,2,4,6,8 et 24 heures, puis dilués avec 900 μl d'eau physiologique contenant 0,85% de chlorure de sodium et 0,1% de tryptone, de manière à obtenir environ 10 à 300 bactéries par plaque gélosée.

On laisse incuber pendant 24 heures, puis l'on compte les colonies qui se sont formées.

On obtient les résultats suivants:

a) nombre de B.stearothermophilus encore viables, par ml de solution (en valeur logarithmique):

| concentration en dicranine (μg/ml) | | | | | | |
|---|---|---|---|---|---|---|
| | Témoin | 10 | 50 | 100 | 150 | 200 |
| 0 heure | 3,699 | 3,699 | 3,724 | 3,699 | 3,711 | 3,699 |
| 2 heures | 4,009 | 3,845 | 3,806 | 3,176 | 2,736 | 2,477 |
| 4 heures | 5,826 | --- | --- | 3,146 | 2,816 | 1,937 |
| 6 heures | 6,975 | 6,628 | 6,439 | 3,263 | 2,766 | 1,544 |
| 8 heures | 7,975 | 8,312 | 7,161 | 3,845 | 2,602 | 1,454 |
| 24 heures | 7,484 | 7,628 | 7,366 | 5,866 | 2,093 | 0,004 |

b) nombre de B.cereus encore viables, par ml de solution (valeur logarithmique):

| concentration en dicranine (μg/ml) | | | | | | |
|---|---|---|---|---|---|---|
| | Témoin | 10 | 50 | 100 | 150 | 200 |
| 0 heure | 3,699 | 3,628 | 3,628 | 3,415 | 3,146 | 2,826 |
| 2 heures | 3,900 | 3,699 | 3,602 | 3,149 | 2,107 | 0,875 |
| 4 heures | --- | --- | --- | 2,924 | 2,049 | 0,041 |
| 6 heures | 6,591 | 6,337 | 5,734 | 2,778 | 1,648 | 0,176 |
| 8 heures | 8,238 | 7,945 | 6,301 | 2,767 | 1,439 | 0,301 |
| 24 heures | 7,544 | 7,544 | 7,0 | 6,071 | 1,124 | 0,041 |

c) nombre de B.subtilis encore viables, par ml de solution (valeur logarithmique):

| concentration en dicranine ($\mu$g/ml) | | | | | | |
|---|---|---|---|---|---|---|
| | Témoin | 10 | 50 | 100 | 150 | 200 |
| 0 heure | 4,301 | 4,301 | 4,301 | 4,000 | 3,954 | 2,204 |
| 2 heures | 4,439 | 4,204 | 3,903 | 3,748 | 3,716 | 1,342 |
| 4 heures | --- | --- | 3,740 | 3,556 | 3,699 | 1,079 |
| 6 heures | 6,954 | 6,851 | 3,732 | 3,665 | 3,618 | 1,021 |
| 8 heures | 8,423 | 7,756 | 3,991 | 3,782 | 3,679 | 0,301 |
| 24 heures | 7,724 | 7,484 | 4,114 | 3,342 | 3,277 | 0,001 |

On constate donc qu'à une concentration de 150 $\mu$g/ml, la dicranine est bactéricide vis-à-vis des bactéries mésophiles B.cereus et B.subtilis. A une concentration de 100 $\mu$g/ml, un effet bactériostatique est observé pendant 4 heures.

Vis-à-vis de la bactérie thermophile B.stearothermophilus, la dicranine est bactéricide à partir de 200 $\mu$g/ml.

A une concentration de 50 à 150 $\mu$g/ml, on observe un effet bactériostatique pendant 4 heures.

**Revendications**

1. Composé chimique constitué par l'acide octadécatriène-9(Z),12(Z), 15(Z)-ynoïque-6 pour son utilisation en tant que substance thérapeutiquement active.

2. Composé chimique selon la revendication 1, pour son utilisation en tant qu'anti-agrégant.

3. Composé chimique selon la revendication 1, pour son utilisation en tant qu'anti-inflammatoire.

4. Composé chimique selon la revendication 1, pour son utilisation en tant qu'anti-bactérien.

5. Composition pharmaceutique comprenant, à titre de substance thérapeutiquement active, l'acide octadécatriène-9(Z),12(Z),15(Z)-ynoïque-6 ainsi qu'un excipient pharmaceutiquement neutre.

6. Composition cosmétique comprenant l'acide octadécatriène-9(Z),12(Z),15(Z)-ynoïque-6.

Office européen
des brevets

**RAPPORT DE RECHERCHE EUROPEENNE**

Numero de la demande

EP 92 11 0344

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | LIPIDS, vol. 9, no. 8, 1974, pages 506-511; B. ANDERSSON et al.: "9,12,15-Octadecatrien-6-ynoic acid, new acetylenic acid from mosses" * Le document en entier * | 1-6 | A 61 K 31/20 |
| | --- | | |
| D,A | BIOCHEMISTRY, vol. 16, no. 7, 1977, pages 1258-1262; J.L. GELLERMAN et al.: "Synthesis and function of 9,12,15-octadecatrien-6-ynoic acid in the moss Ceratodon purpureus" * Le document en entier * | 1-6 | |
| | ----- | | |

|  |  |
|---|---|
| | **DOMAINES TECHNIQUES RECHERCHES (Int. Cl.5)** |
| | A 61 K |

**Le présent rapport a été établi pour toutes les revendications**

| Lieu de la recherche | Date d'achèvement de la recherche | Examinateur |
|---|---|---|
| LA HAYE | 19-08-1992 | GOETZ G. |

EPO FORM 1503 03.82 (P0402)